# EUROPEAN PATENT APPLICATION

(11) **EP 3 354 195 A1**
(43) Date of publication of application: **01.08.2018**
(21) Application number: 17206760.5
(22) Date of filing: 12.12.2017
(51) Int. Cl.: A61B 5/021

(54) **PRESSURE SENSOR AND BLOOD PRESSURE MEASUREMENT DEVICE**

(30) Priority: 25.01.2017 TW 106103044
(71) Applicant: Maisense Inc., Zhubei City 30273 (TW)
(72) Inventor: Lee, Yung-Pin, 30273 Zhubei City (TW); Lee, Min-Hao, 30273 Zhubei City (TW)
(74) Representative: dompatent von Kreisler Selting Werner - Partnerschaft von Patent- und Rechtsanwälten mbB

(57) **Abstract**

A pressure sensor and a blood pressure measurement device with the pressure sensor are disclosed. The pressure sensor includes a case and a pressure sensing element. Two opposite sides of the case have a concave portion and a convex portion, respectively. A recessed space is formed in the concave portion. The pressure sensing element is disposed on the case. When the convex portion pushes the pressure sensing element, the pressure sensing element is deformed and protruded toward the recessed space. The pressure sensor has a higher detection sensibility.

## Description

### BACKGROUND

### Technical Field

The present disclosure relates to a pressure sensor and a blood pressure measurement device.

### Related Art

The blood pressure is one of important indicators for the observation of the individual's health condition. The abnormal value of the blood pressure, either the too-high or too-low value, implies that a problem of the individual's physiological condition, especially the cardiovascular disease that is difficult to be observed from the outlook of the individual, arises. Because the elder's body has the aging function, there is a high risk of suffering from the cardiovascular disease, and it is a very important work to regularly measure the blood pressure of the elder so that the disease can be detected and treated early. However, the modem human beings have the high life pressure, and the irregular three meals. Thus, the group of persons whose blood pressures need to be regularly monitored has the decreasing age.

A conventional sphygmomanometer includes a cuff, a host and a rubber tube. Upon measurement of the blood pressure, the host controls the inflating pressurization and the deflating depressurization on the cuff through the rubber tube 13, and calculates the blood pressure based on the pressure fluctuation value, which is sensed by the pressure sensor contained in the cuff.

However, the conventional sphygmomanometer tends to make the subject feel uncomfortable in the pressurization and depressurization processes, and needs a longer measurement time, so that the subject's measurement desire is low, and it is disadvantageous to the long-term use. In addition, regarding the portable property, the sizes of the host and the cuff are too large, and are disadvantageous to the monitoring of the blood pressure at any time. In addition, if the cuff is not properly mounted and fixed on the body, the vessel pulse signal may not be precisely measured, or the detecting sensitivity may become poor. This situation can cause the error or failure of the detection of the blood pressure measurement.

### SUMMARY

An objective of the disclosure is to provide a pressure sensor and a blood pressure measurement device having a structural design for providing a higher detection sensitivity.

The present disclosure provides a pressure sensor, which includes a case and a pressure sensing element. Two opposite sides of the case have a concave portion and a convex portion, respectively. A recessed space is formed in the concave portion. The pressure sensing element is disposed on the case. When the convex portion pushes the pressure sensing element, the pressure sensing element is deformed and protruded toward the recessed space.

In one embodiment, the pressure sensing element is a strain gauge or a piezoelectric element.

In one embodiment, the concave portion, the convex portion and the pressure sensing element are at least partially overlapped in a projection direction.

In one embodiment, the convex portion has an arc surface, and the pressure sensing element fits to the arc surface.

In one embodiment, the concave portion has an arc surface, and the pressure sensing element fits to the arc surface.

In one embodiment, the pressure sensing element is disposed inside the case and located between the concave portion and the convex portion.

In one embodiment, a side wall of the concave portion has a stepped shape.

In one embodiment, a plurality of ribs are disposed inside the concave portion, and the ribs are intersected to form a plurality of the recessed spaces.

The present disclosure also provides a blood pressure measurement device, which includes the above-mentioned pressure sensor and a first electrode. The first electrode is disposed on a surface of the case away from the convex portion.

In one embodiment, the concave portion is disposed inside the case and located between the concave portion and the surface.

In one embodiment, the blood pressure measurement device is operated on a body portion of a user having a blood vessel, and when measuring a blood pressure, the first electrode, the concave portion, the pressure sensing element, the convex portion and the blood vessel are at least partially overlapped in a projection direction.

In one embodiment, when the user touches the first electrode, the user pushes the convex portion to fit on the body portion.

In one embodiment, a finger of the user is contact with the first electrode, the case has a recess corresponding to a shape of the finger, and the first electrode is disposed in the recess.

In one embodiment, the blood pressure measurement device further includes a second electrode disposed adjacent to the convex portion and electrically connected to the first electrode. When measuring the blood pressure, the second electrode is contacted with the body portion.

In one embodiment, the blood pressure measurement device further includes a calibration electrode, a processing unit, and a storage unit. The calibration electrode is disposed on the case, and the processing unit is disposed in the case. The storage unit is disposed in the case and signally connected to the processing unit. The storage unit stores a program instruction, and when the processing unit executes the program instruction, the processing unit performs following steps of: obtaining a first calibration value, wherein the first calibration value is obtained by calculating a first measurement result of the calibration electrode and the first electrode; obtaining a second calibration value, wherein the second calibration value is obtained by calculating a second measurement result of the calibration electrode and the first electrode; and calibrating measured values of the first electrode and the second electrode according to the first calibration value and the second calibration value.

In one embodiment, when the pressure sensing element is pushed by the convex portion and deformed and protruded toward the recessed space, the configuration of the concave portion and the convex portion can increase a margin for the deformation of the pressure sensing element.

In one embodiment, when the disclosure is applied to measure pulses, the pressure sensing element is deformed accompanying with the beat of the pulse, and the deformations of the pressure sensing element can be rapidly recovered.

As mentioned above, in the pressure sensor and blood pressure measurement device of the disclosure, the pressure sensing element is configured to be pushed by the convex portion and deformed and protruded toward the recessed space, so that the pressure sensor and blood pressure measurement device of the disclosure can have a higher detecting sensitivity. In one embodiment of the disclosure, the structural design of the blood pressure measurement device can calculate the blood pressure according to the sensing signals of two electrodes and the pressure sensing element, and the host and cuff for pressurization and depressurization are not needed. Thus, the size can be advantageously decreased so that the blood pressure measurement device can be easily carried, and the user's desire of monitoring the blood pressure can be enhanced. In addition, in the structural design, the pressure sensing element can be pushed by the convex portion and deformed and protruded toward the recessed space of the concave portion. Compared to the design without the concave portion, the pressure sensor and the blood pressure measurement device of this disclosure can have a higher detecting sensitivity.

### BRIEF DESCRIPTION OF THE DRAWINGS

The embodiments will become more fully understood from the detailed description and accompanying drawings, which are given for illustration only, and thus are not limitative of the present invention, and wherein:
FIGS. 1A and 1B are schematic perspective views of a pressure sensor according to an embodiment of the disclosure;
FIG. 1C is an exploded view of the pressure sensor of FIG. 1A;
FIGS. 2A to 2G are schematic diagrams showing pressure sensors of different aspects of the disclosure;
FIGS. 3A and 3B are schematic perspective views of a blood pressure measurement device according to an embodiment of the disclosure;
FIG. 3C is a system block diagram of the blood pressure measurement device of FIG. 3A;
FIG. 4A is a schematic diagram showing the operation of the blood pressure measurement device of FIG. 3A;
FIGS. 4B to 4C are partial sectional views of the blood pressure measurement device of FIG. 3A;
FIGS. 5A to 5C are schematic diagrams showing the components of the blood pressure measurement device; and
FIG. 6 is a schematic diagram showing the detecting sensitivities of different blood pressure measurement devices.

### DETAILED DESCRIPTION OF THE INVENTION

The embodiments of the invention will be apparent from the following detailed description, which proceeds with reference to the accompanying drawings, wherein the same references relate to the same elements.

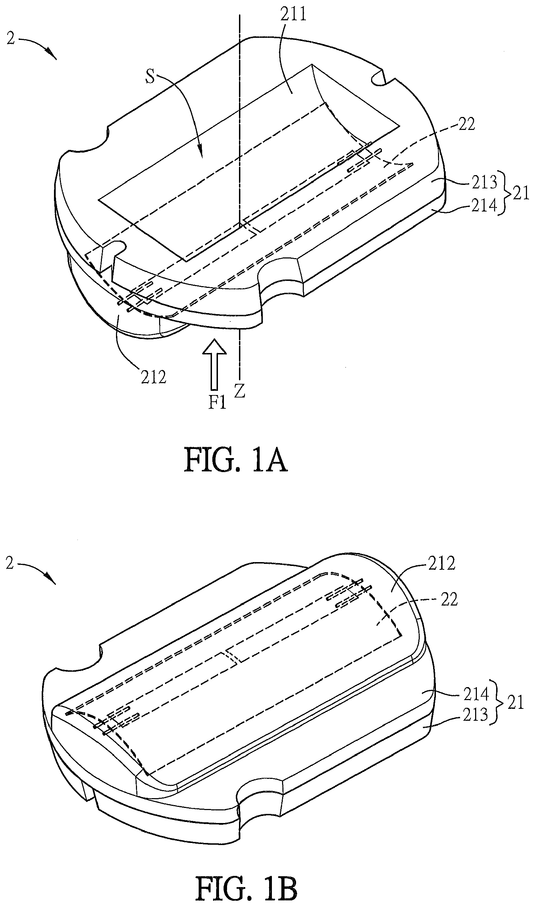

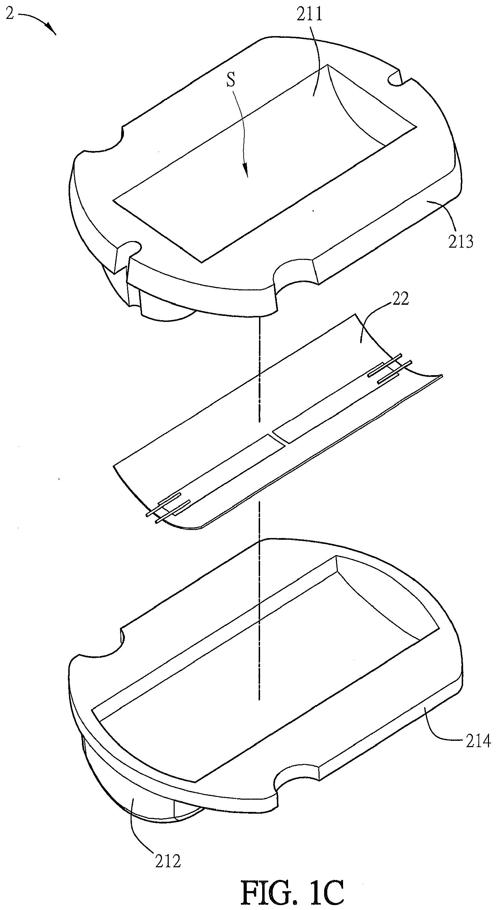

FIGS. 1A and 1B are schematic perspective views of a pressure sensor 2 according to an embodiment of the disclosure, and FIG. 1C is an exploded view of the pressure sensor 2 of FIG. 1A.

Referring to FIGS. 1A to 1C, the pressure sensor 2 includes a case 21 and a pressure sensing element 22. The case 21 is configured to accommodate and fix the electronic elements of the pressure sensor 2. The case 21 has a concave portion 211 and a convex portion 212 disposed at two opposite sides of the case 21, respectively. A recessed space S is formed in the concave portion 211. As shown in FIG. 1A, the concave portion 211 is a recessed slot having a recessed space S, and is disposed on the top surface of the case 21. The convex portion 212 is protruded toward a direction away from the concave portion 211, and is disposed on the bottom surface of the case 21. The materials of the concave portion 211 and the convex portion 212 can be the same as or different from other portions of the case 21. This disclosure is not limited. In some embodiments, the case 21 is integrally formed as a single piece. In some embodiments, the case 21 can be assembled by a plurality of separated parts. In some embodiments, the case 21 can be made of an elastic material (e.g. silica gel or rubber), so that it can have deformation during the measurement.

The pressure sensing element 22 is disposed on the case 21, and it can be a strain gauge or a piezoelectric element. In this embodiment, the pressure sensing element 22 includes two strain gauges arranged in parallel. The strain gauge is composed of a piezoresistive material (e.g., a metal sheet) and an insulating substrate. In one application example, the pressure sensor 2 is used in a blood pressure meter. When measuring the blood pressure, the convex portion 212 of the pressure sensor 2 contacts, for example, the wrist of the user and senses the pulsation of the radial artery within the wrist so as to deform the strain gauge (the pressure sensing element 22). The strain gauge deformation causes the variation of the resistance value, and then the pressure value can be calculated according to the variation of the resistance value. The calculated pressure value is then converted into the pulse signal. The two ends of the pressure sensing element 22 can be connected to two conductive wires, respectively. After applying electricity to the two conductive wires, a voltage meter or a current meter can be provided to measure the voltage value or the current value so as to obtain the variation of the resistance value, followed by the calculation of the pressure value and the pulse signal.

When the convex portion 212 pushes the pressure sensing element 22, the pressure sensing element 22 is deformed and protruded toward the recessed space S. In this embodiment, the pressure sensing element 22 is disposed inside the case 21 and located between the concave portion 211 and the convex portion 212. As shown in FIG. 1C, the case 21 of this embodiment is composed of two parts, including a first case part 213 and a second case part 214. The top surface of the first case part 213 is recessed to form the concave portion 211, and the bottom surface of the second case part 214 protrudes to form the convex portion 212. The pressure sensing element 22 is disposed between the first case part 213 and the second case part 214. The concave portion 211, the convex portion 212 and the pressure sensing element 22 are at least partially overlapped in a projection direction Z (as shown in FIG. 1A). The shapes of the concave portion 211, the convex portion 212 and the pressure sensing element 22 are corresponding to each other and are all curved shapes toward the same side. In some embodiments, the thickness of the concave portion 211 of the pressure sensor 2 is between 0.5 mm and 1.4 mm.

Upon measurement, as shown in FIG. 1A, the pressure sensor 2 is pushed downwardly, so that when the convex portion 212 contacts the body, a force F1 is applied to the convex portion 212 so as to deform the convex portion 212. Since the case 21 is configured with the concave portion 211, the pressure sensing element 22 is deformed and protruded toward the recessed space S when the convex portion 212 pushes the pressure sensing element 22. Compared to the design without the concave portion, this embodiment configures the recessed space S in the concave portion 211 for increasing the deformation amount of the pressure sensing element 22, thereby improving the detecting sensitivity. In this embodiment, the increased deformation amount of the pressure sensing element 22 (e.g. after being pushed by the convex portion 212) is caused by the increased length or the larger curve of the pressure sensing element 22. In addition, after the convex portion 212 is pushed and protruded toward the recessed space S, the convex portion 212 can rapidly bounce back, and the concave portion 211 and the pressure sensing element 22 can be recovered to the original status.

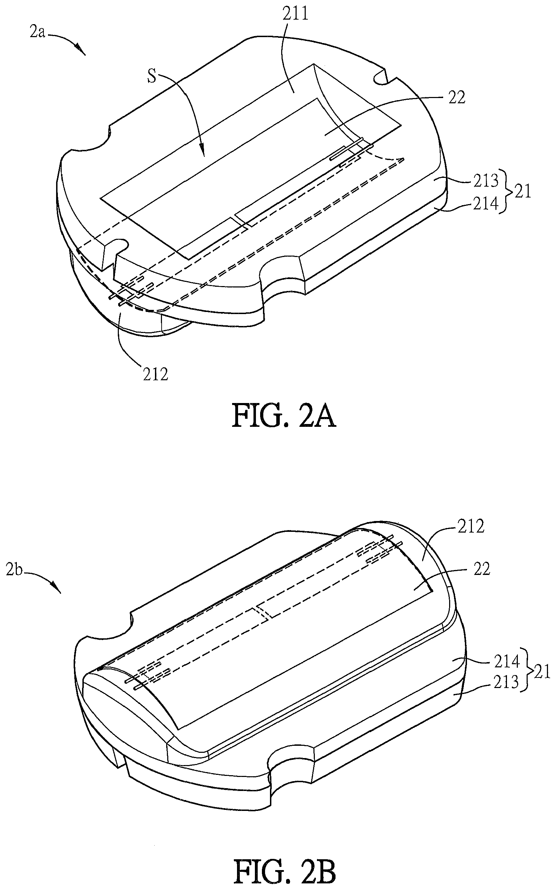

FIGS. 2A to 2G are schematic diagrams showing pressure sensors 2a∼2g of different aspects of the disclosure.

Different from the above embodiment having the pressure sensing element 22 disposed inside the case 21, the pressure sensing element 22 can be configured at other places. As shown in FIG. 2A, the pressure sensing element 22 of this aspect is disposed in the concave portion 211. In this aspect, the concave portion 211 has an arc shape, and the pressure sensing element 22 is fit to an arc surface of the concave portion 211. Alternatively, as shown in FIG. 2B, the convex portion 212 of this aspect has an arc shape, and the pressure sensing element is fit to an arc surface of the convex portion 212.

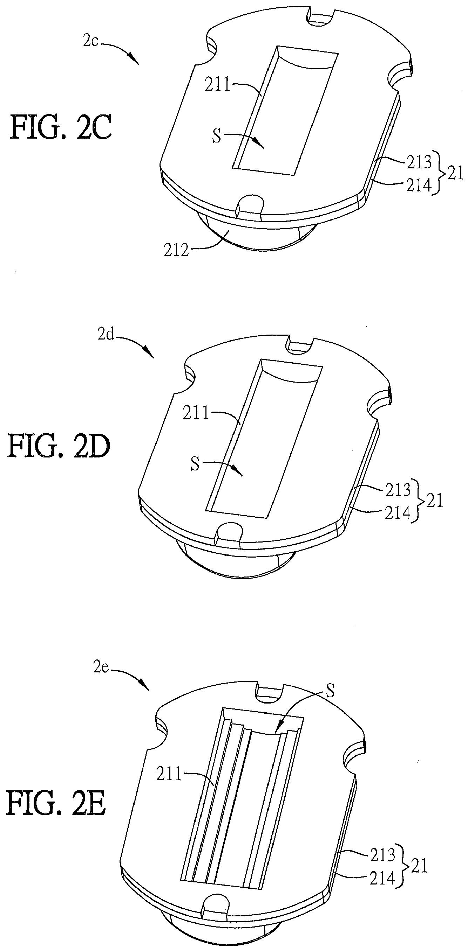

As shown in FIG. 2C, different from the pressure sensor 2 of FIG. 2A, the concave portion 211 of the pressure sensor 2c has only one arc surface at the bottom.

Different from the pressure sensor 2c of FIG. 2C, in the pressure sensor 2d as shown in FIG. 2D, the length of the concave portion 211 is longer so that the recessed space S of the concave portion 211 is larger. Accordingly, when the concave portion 212 pushes the pressure sensing element 22, the pressure sensor 2d can provide a larger space for the deformed pressure sensing element 22, thereby further improving the detecting sensitivity.

Different from the pressure sensor 2 of FIG. 2A, as shown in FIG. 2E, the two opposite side walls of the concave portion 211 of the pressure sensor 2e are configured with stepped shapes. This configuration can provide a larger deformation space for the sensor, and allow the sensor to have a better bounce force.

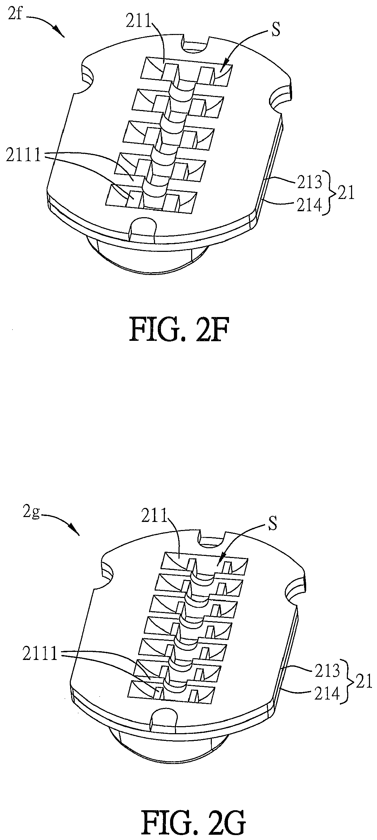

Different from the pressure sensor 2 of FIG. 2A, as shown in FIG. 2F, the concave portion 211 of the pressure sensor 2f is configured with a plurality of ribs 2111, which are intersected to form a plurality recessed spaces S. In this aspect, a part of the ribs 2111 extend along one direction, and the other part of the ribs 2111 extend along another direction. Thus, the ribs 2111 can intersect to form a plurality recessed spaces S. Since the concave portion 211 has a plurality of recessed spaces S, the sensor can have better bounce force. Besides, the ribs 2111 can support the appearance of the sensor and prevent the undesired subsidence.

Different from the pressure sensor 2f of FIG. 2F, as shown in FIG. 2G, the ribs 2111 disposed in the concave portion 211 of the pressure sensor 2g are thinner, so that the amount of the recessed spaces S formed by the intersected ribs 2111 is greater than that of the pressure sensor 2f. In addition, since the ribs 2111 of the pressure sensor 2 are thinner, the pressure sensor 2g can provide a larger deformation space than the pressure sensor 2f. This configuration can improve the detecting sensitivity.

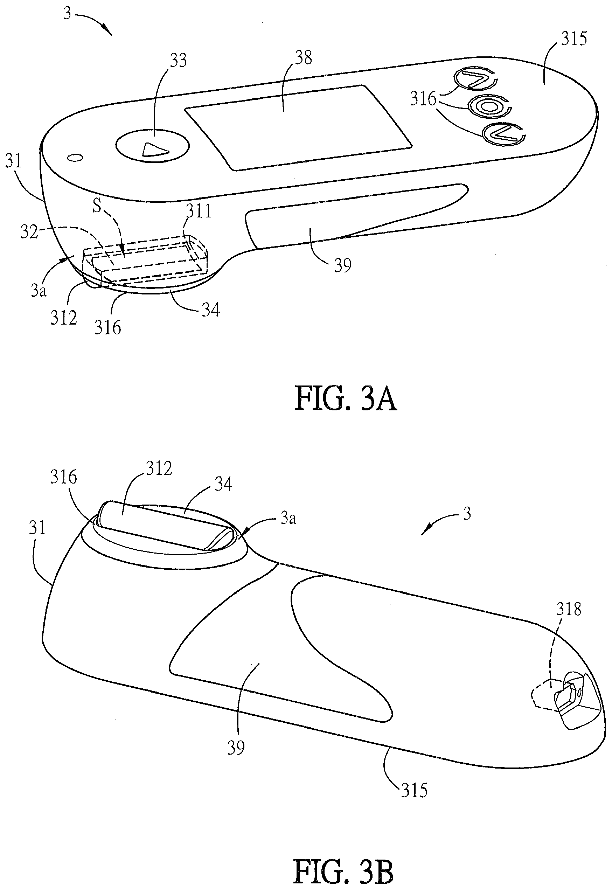

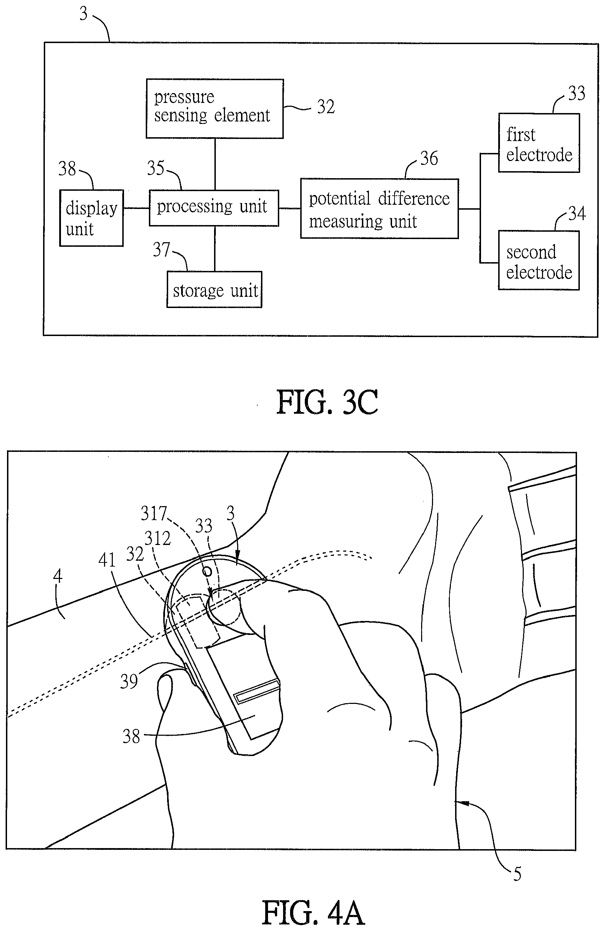

FIGS. 3A and 3B are schematic perspective views of a blood pressure measurement device 3 according to an embodiment of the disclosure, and FIG. 3C is a system block diagram of the blood pressure measurement device 3 of FIG. 3A.

Referring to FIGS. 3A to 3C, the above-mentioned concave portion, pressure sensing element and convex portion of the pressure sensor are applied to the blood pressure measurement device 3. The blood pressure measurement device 3 includes a pressure sensor 3a and a first electrode 33. The pressure sensor 3a includes a case 31 and a pressure sensing element 32. In addition, the blood pressure measurement device 3 further includes a second electrode 34, a processing unit 35, a potential difference measuring unit 36, a storage unit 37, and a display unit 38.

The case 31 is configured to accommodate and fix the electronic elements of the blood pressure measurement device 3. The case 31 has includes two opposite surfaces 315 and 316. When viewed from the operation angle of the user, the surface 315 is an upper surface close to the user, and the surface 316 is a lower surface away from the user. The surface 315 or the surface 316 of the case 31 has notches so that the electrodes and the elements can be firmly fixed onto the surfaces and electrically connected to the other elements inside the case 31. In addition, the notches are also provided so that each of the electrodes and the elements can be partially exposed outside the case 31 to achieve the object of contacting the part of the body or displaying the results.

The two opposite sides of the case 31 are configured with a concave portion 311 and a convex portion 312, respectively, and a recessed space S is formed in the concave portion 311. As shown in FIG. 3A, the concave portion 311 is disposed inside the case 31 and located between the convex portion 312 and the surface 315. In this embodiment, the concave portion 311 is a recessed slot having a recessed space S, and the convex portion 312 is protruded toward a direction away from the concave portion 311 and is disposed on the surface 316. The materials of the concave portion 311 and the convex portion 312 can be an elastic material (e.g. silica gel or rubber), so that they can easily have deformation during the measurement.

Similar to the above-mentioned pressure sensing element 22, the pressure sensing element 32 of this embodiment includes two strain gauges arranged in parallel. As shown in FIG. 4A, the blood pressure measurement device 3 is placed and operated on the left-hand's wrist, so the convex portion 312 contacts the left-hand's wrist of the user and senses the pulsation of the radial artery within the wrist upon measurement of the blood pressure. When the convex portion 312 pushes the strain gauge (the pressure sensing element 32), the pressure sensing element 32 is deformed and protruded toward the recessed space S. The strain gauge deformation causes the variation of the resistance value, and then the microprocessor can calculate the pressure value according to the variation of the resistance value. The obtained variation of the continuous pressure values is transmitted to the processing unit, and then converted into the pulse signal. In other embodiments of the disclosure, the pressure sensing element 32 may also be an element made of a piezoelectric material. The technical features of the pressure sensor 3a of the blood pressure measurement device 3 can be referred to the above-mentioned pressure sensor 2, so detailed descriptions thereof will be omitted.

The first electrode 33 and the display unit 38 are disposed on the surface 315 of the case 31, and the second electrode 34 is located adjacent to the convex portion 312 and disposed on the surface 316 of the case 31. In this embodiment, the second electrode 34 is disposed around the convex portion 312 and electrically connected to the first electrode 33. The first electrode 33 and the second electrode 34 are paired electrodes, wherein one of them is a positive electrode, and the other of them is a negative electrode. Both of the first electrode 33 and the second electrode 34 are electrically connected to the potential difference measuring unit 36. The first electrode 33 and the second electrode 34 form one set of leads, and contact different parts of the user's body, respectively. When the heart is performing the depolarization activity, the potential difference measuring unit 36 can measure one set of potential differences between the electrodes, and the processing unit 35 converts the set of potential differences into an electrocardio signal. In this embodiment, the first electrode 33 contacts the user's right limb (particularly the right-hand's index finger as shown in FIG. 4A), and the second electrode 34 contacts the user's left limb (particularly the left-hand's wrist) to form a loop. In this embodiment, two electrodes are used to contact and measure user's left and right limbs, so the measured electrocardio signal may be referred to as a limb lead electrocardio signal. Specifically speaking, the limb lead electrocardio signal is a first limb lead (lead I) serving as a single lead electrocardio signal. In this embodiment, the electrocardio signal is an electrocardiogram (EGC) signal. However, it is to be noted that, in other embodiments of the disclosure, the first electrode 33 and the second electrode 34 may also contact other parts of the body, such as the left and right legs, the part near the rib of the trunk or the part near the armpit or the likes, to measure the electrocardio signals of other leads. The disclosure is not restricted thereto.

In this embodiment, the first electrode 33, the concave portion 311, the pressure sensing element 32, and the convex portion 312 are disposed at upper and lower positions opposite each other in this embodiment. If viewed at the angle of the user, the first electrode 33 is disposed above the concave portion 311, the pressure sensing element 32, and the convex portion 312. When the size of the first electrode 33 is slightly smaller than that of the pressure sensing element 32 or the convex portion 312 and if the projection is made in a projection orientation perpendicular to the surface of the first electrode 33, then the projection of the first electrode 33 certainly falls within the projection of the pressure sensing element 32 or the convex portion 312. On the contrary, if the size of the first electrode 33 is greater than the pressure sensing element 32 or the convex portion 312 and the projection is similarly made in the projection orientation, then the projection of the first electrode 33 covers the projection of the pressure sensing element 32 or the convex portion 312. In addition, if the size of the first electrode 33 approaches that of the pressure sensing element 32 or the convex portion 312 but they have different shapes, and the projection is similarly made in the projection orientation, then the projections of the first electrode 33 and the pressure sensing element 32 or the convex portion 312 may partially non-overlap but anyway may partially overlap with one another.

The processing unit 35 and the storage unit 37 are individually accommodated in the case 31. Referring to FIG. 3C, the processing unit 35 is electrically connected to the potential difference measuring unit 36, the storage unit 37, the display unit 38 and the pressure sensing element 32. The storage unit 37 stores program instructions and data, and the processing unit 35 accesses the storage unit 37 to obtain the program instructions and data, required for processing, and to perform data computation according to the program instructions to control other units to operate.

While the processing unit 35 calculates the electrocardio signal through the potential difference measuring unit 36, and calculates the pulse signal through the pressure sensing element 32, the times of getting the two signals are also recorded, so that the pulse transmission time (PTT) can be calculated according to the time difference. In detail, the PTT is the difference between the time of appearance of the R wave (corresponding to the medium term of depolarization of the ventricular) of the electrocardio signal being judged in one heartbeat, and the time of occurrence of the pulsation of the radial artery measured on the to-be-measured part of the body (the left-hand's wrist in this embodiment). In other words, the PTT is the time duration when the pulse generated in one heartbeat travels from the heart to the left-hand's wrist. The distance from the heart to the left-hand's wrist may be a predetermined value, or may be obtained after the height of the user is manually inputted and adjusted according to a parameter. Then, the distance is divided by the PTT to obtain the pulse wave velocity (PWV), which is the velocity of the pulse, which is generated by the systole and reaches the left-hand's wrist. Thereafter, the processing unit 35 can calculate the user's blood pressures, including the systolic pressure and the diastolic pressure, according to the PWV through the algorithm. In this embodiment, the processing unit 35 may transmit the measured result to the display unit 38 for display. Of course, in other embodiments, the blood pressure measurement device 3 may have a communication unit, which transmits the measured result to a smart phone or a tablet computer for display through Bluetooth, 3G or 4G mobile communication technology or wireless communication method (e.g., Wi-Fi). This disclosure is not restricted thereto.

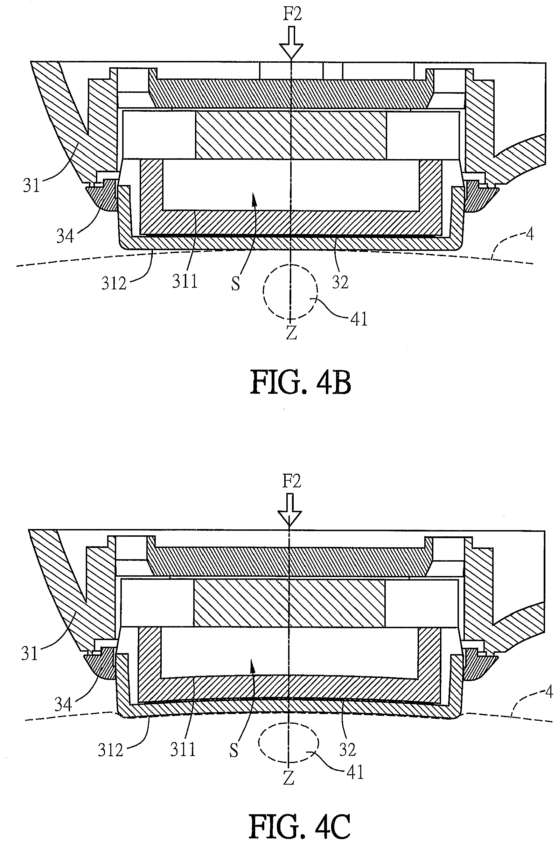

FIG. 4A is a schematic diagram showing the operation of the blood pressure measurement device 3 of FIG. 3A, and FIGS. 4B to 4C are partial sectional views of the blood pressure measurement device 3 of FIG. 3A.

Referring to FIG. 4A, when the user's hand holds the blood pressure measurement device 3, the user's limb 5, preferably the finger (the right-hand's index finger in this embodiment), can easily touch the first electrode 33 due to the mechanism design. Preferably, the case 31 of the blood pressure measurement device 3 has a recessed slot 317 corresponding to the shape of the finger, and the first electrode 33 is disposed in the recessed slot 317. Upon measurement of the blood pressure, the recessed slot 317 has the function of prompting the user to use the hand to contact or press to operate. This design can further enhance the effect of instinctively operating the blood pressure measurement device, and further assist in fixing the position of the finger so that the user can exert the force more easily.

When the user places the blood pressure measurement device 3 on the to-be-measured part 4 of the body for the preparation of measurement, the first electrode 33 is disposed above the concave portion 311, the pressure sensing element 32 and the convex portion 312, and the convex portion 312 is aligned to the blood vessel 41 and disposed above the blood vessel 41. If the projection is made in a projection orientation perpendicular to the surface of the first electrode 33, then the projections of the first electrode 33, the concave portion 311, the pressure sensing element 32, the convex portion 312 and the blood vessel 41 are at least partially overlapped in the same projection direction Z.

Referring to FIGS. 4A to 4C, upon the measurement of the blood pressure, when the right hand's finger of the user touches the first electrode 33, the downward pressing force generated by a gravity force exerted to the limb 5, the downward pressing force generated when the user becomes aware of the measurement and instinctively forces the finger to tightly press the first electrode 33, or the combination of the two downward pressing forces can be provided to cause an external force F 2. The external force F2 exerted on the first electrode 33 is also exerted on the convex portion 312 with the similar magnitude, so that the convex portion 312 is firmly and tightly pressed upon the left-hand's wrist and deformed. Accordingly, the pressure sensing element 32 can clearly sense the pulsation of the blood vessel 41 and protrude toward the recessed space S, thereby precisely obtaining the pulse signal. More particularly, it is possible to overcome the problem of the measurement failure caused when the convex portion 312 is not tightly pressed upon the wrist and the pulsation of the blood vessel 41 cannot be sensed.

In this embodiment, the second electrode 34 is mounted on the periphery of the convex portion 312. So, when the user utilizes the right hand's finger to touch the first electrode 33, he or she can make the convex portion 312 be firmly and tightly press upon the left-hand's wrist at his/her convenience, while the second electrode 34 may also be tightly pressed upon the left-hand's wrist to ensure the formation of the loop and to ensure that the electrocardio signal can be obtained.

In the mechanism design of the blood pressure measurement device 3, because the first electrode 33, the concave portion 311, the pressure sensing element 32, the convex portion 312, and the blood vessel 41 at least partially overlap with one another in the projection orientation Z, an instinctive operation is caused. First, this is because that the limb 5 is kept in contact with the first electrode 33 from the time when the hand holds the blood pressure measurement device 3 to the time of starting the measurement without any movement or adjustment of the position, and that the operation is similar to the diagnosis of the pulse using the finger. Second, when viewed at the angle where the force is exerted onto the first electrode 33, the same force can be provided to the convex portion 312 without additionally increasing the force. Third, when viewed at the angle where the force is exerted to make the convex portion 312 be firmly pressed upon the part 4 of the body, the same force can make the limb 5 and the first electrode 33 be tightly pressed upon each other without additionally increasing the force. As shown in FIG. 4C, when the convex portion 312 pushes the pressure sensing element 32, the pressure sensing element 32 is deformed and protruded toward the recessed space S. In other embodiments of the disclosure, the user's limb 5 may be the index finger of the left or right hand, and the part 4 of the body may be any part of the body through which the blood vessel passes and the pulse can be easily obtained, wherein the part may be near the wrist, the arm, the leg, the neck or the rib of the trunk, for example. The disclosure is not restricted thereto.

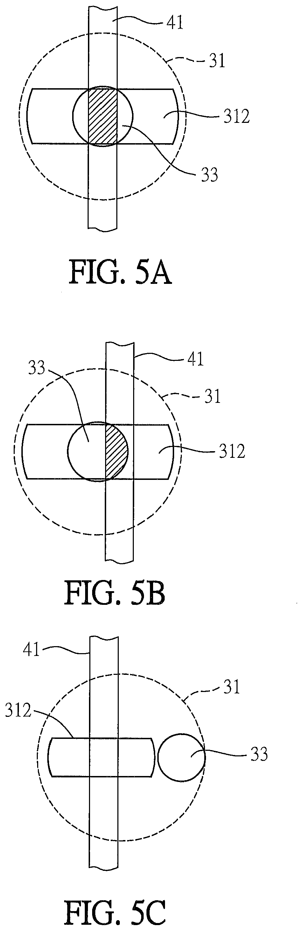

FIGS. 5A to 5C are schematic views showing elements of the blood pressure measurement device 3, and are provided for describing the projecting states of the first electrode 33, the convex portion 312 and the blood vessel 41 when being projected in the projection orientation Z. FIG. 5A shows that the first electrode 33, the convex portion 312 and the blood vessel 41 of the blood pressure measurement device 3 completely overlap with one another in the projection orientation Z. FIG. 5A shows that the first electrode 33, the convex portion 312 and the blood vessel 41 of the blood pressure measurement device 3 partially overlap with one another in the projection orientation Z. FIG. 5C shows that the first electrode 33, the convex portion 312 and the blood vessel 41 of the blood pressure measurement device 3 are not overlapped with one another in the projection orientation Z.

As shown in FIG. 5A, in this embodiment, when the first electrode 33, the convex portion 312 (the pressure sensing element, the concave portion) and the blood vessel 41 completely overlap with one another in the projection orientation Z, the user uses a limb 5 to exert an external force to contact or press the first electrode 33, and the blood pressure measurement device 3 may also tightly and firmly press the convex portion 312 upon the wrist through the external force at the same time. That is, the convex portion 312 is disposed above the blood vessel 41, so that the effect of fixing the blood pressure measurement device 3 is obtained, and it is also advantageous to the sensing of the pulsation of the blood vessel 41 and the enhancement of the accuracy of the pulse signal.

As shown in FIG. 5B, even when a little deviation of the placement position of the blood pressure measurement device 3 is present (e.g., the device 3 is not directly placed above the blood vessel 41 of the wrist but is slightly biased leftward or rightward), the effect that the first electrode 33, the convex portion 312 (the pressure sensing element, the concave portion), and the blood vessel 41 at least partially overlap with one another in the projection orientation can be maintained as long as the convex portion 312 still faces toward the blood vessel 41. At this time, the external force F2 exerted from the limb 5 to the first electrode 33 still can be provided to the convex portion 312 with the same magnitude to achieve the effect of tightly pressing the wrist at the user's convenience without deliberately increasing the force to overcome the problem of the deviation of the placement position.

On the contrary, FIG. 5C shows the assumed condition where the first electrode 33, the convex portion 312 (the pressure sensing element, the concave portion), and the blood vessel 41 of the blood pressure measurement device 3 do not at least partially overlap with each other in the projection orientation. In this case, the external force exerted from the user to the first electrode 33 cannot assist in pressing the convex portion 312 firmly upon the wrist, so that the pulse signal cannot be measured or the measured pulse signal is not clear. The condition of FIG. 5C becomes more obvious in the watch-type blood pressure measurement device because the convex portion 312 and the first electrode 33 are disposed on different peripheral positions of the wrist. Even if the external force exerted on the first electrode 33 is increased, the force required to press the convex portion 312 cannot be provided because of the components of the force. In addition, because the force has to be increased, the user may feel the inconvenience of operation, and this is not an instinctive operation.

Referring to FIGS. 3A and 3B, the blood pressure measurement device 3 further includes a calibration electrode 39. The calibration electrode 39 may be disposed on a left side or a right side of a case 31, and is electrically connected to the potential difference measuring unit 36 (not shown). Upon operation, with reference to FIG. 4A, the calibration electrode 39 contacts another limb of the user, such as the thumb and the middle finger of the right hand. When the processing unit 35 executes the program instructions stored in the storage unit 37, the potential difference measuring unit 36 measures the potential difference between the calibration electrode 39 and the first electrode 33 at least two times within a predetermined period of time. The two measured results will be transmitted to the processing unit 35, and the processing unit 35 calculates a first calibration value and a second calibration value according to the measured results. Next, the processing unit 35 further calibrates an electrocardio signal, obtained by the measurement of the first electrode 33 and the second electrode 34, according to an average of the first calibration value and the second calibration value. The calibration is based on the average of the calibration values to correct or remove the extreme values (e.g., the maximum and minimum value) of the electrocardio signal to obtain the electrocardio signal with the higher credibility.

In addition, the case 31 of the blood pressure measurement device 3 of this embodiment has arced front and rear ends. The arced rear end of the case 31 can make the user easily hold the blood pressure measurement device 3, and naturally and instinctively use the limb 5 to touch the first electrode 33 and the calibration electrode 39. Thus, upon measurement of the blood pressure, the user can fix the blood pressure measurement device 3 onto the part 4 of the body in an instinctive operation manner, and then contact or even press the first electrode 33 to make the convex portion 312 firmly and tightly press upon the part above the blood vessel.

In addition, the blood pressure measurement device 3 of this embodiment may further include an input module 319 and a connection port 318. The user can use the input module 319 to input the personal physiological information (including, for example but without limitation to, the height) to adjust the calculation parameter and enhance the measurement accuracy. The user can obtain the personal blood pressure through the display unit 38, and may also utilize the connection port 318 to transmit the blood pressure to the electronic device to record the personal blood pressure.

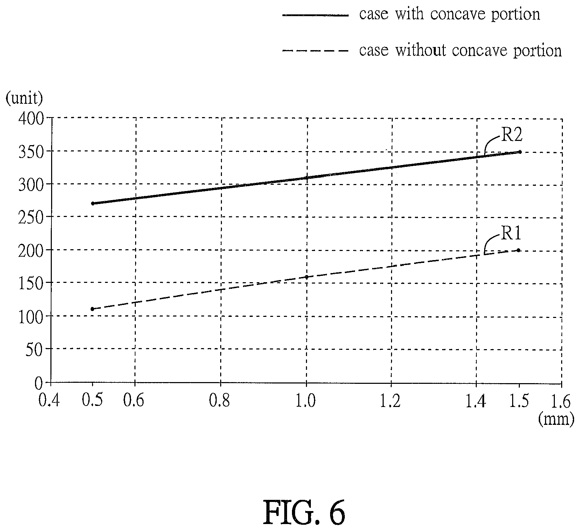

FIG. 6 is a schematic diagram showing the detecting sensitivities of different blood pressure measurement devices. The measuring results shown in FIG. 6 are obtained by two blood pressure measurement devices with different structural designs under the same measurement conditions. Herein, the broken line R1 shows the measuring result obtained by the blood pressure measurement device having a case configured without the concave portion, and the broken line R2 shows the measuring result obtained by the above-mentioned blood pressure measurement device 3.

Referring to FIG. 6, the horizontal coordinate represents a descending distance of the blood pressure measurement device upon measurement, and the vertical coordinate represents a resistance signal outputted by the blood pressure measurement device. As shown in FIG. 6, under the same descending distance (e.g. 0.5 mm), the measuring value of the broken line R1 is 110, and the measuring value of the broken line R2 is 270. In addition, when the descending distance is 1.0 mm, the measuring value of the broken line R1 is 160, and the measuring value of the broken line R2 is 310. When the descending distance is 1.5 mm, the measuring value of the broken line R1 is 200, and the measuring value of the broken line R2 is 350. Obviously, the measuring values of the blood pressure measurement device 3 are always greater than those of the blood pressure measurement device having a case configured without the concave portion. Accordingly, the blood pressure measurement device 3 can have a higher detecting sensitivity (about 2 times).

As mentioned above, in the pressure sensor and blood pressure measurement device of this disclosure, the pressure sensing element is disposed in the case and can be deformed and protruded into the recessed space when being pushed by the convex portion. Accordingly, the disclosure has the following two advantages. First, when the convex portion is deformed, the other parts of the case do not block the deformation of the pressure sensing element, and the configurations of the convex portion and the concave portion can provide a larger space for accommodating the deformation of the pressure sensing element. Second, since the thicknesses of the convex portion and the pressure sensing element are very thin, the pressure sensing element can be still pushed by the convex portion and deformed and protruded toward the recessed space even if the beating amplitude of the pulse is very small. Thus, the pressure sensing element can move accompanying with the beat of the pulse, and rapidly bounced back and recovered after the deformation.

To sum up, in the pressure sensor and blood pressure measurement device of the disclosure, the pressure sensing element is configured to be pushed by the convex portion and deformed and protruded toward the recessed space, so that the pressure sensor and blood pressure measurement device of the disclosure can have a higher detecting sensitivity. In one embodiment of the disclosure, the structural design of the blood pressure measurement device can calculate the blood pressure according to the sensing signals of two electrodes and the pressure sensing element, and the host and cuff for pressurization and depressurization are not needed. Thus, the size can be advantageously decreased so that the blood pressure measurement device can be easily carried, and the user's desire of monitoring the blood pressure can be enhanced. In addition, in the structural design, the pressure sensing element can be pushed by the convex portion and deformed and protruded toward the recessed space of the concave portion. Compared to the design without the concave portion, the pressure sensor and the blood pressure measurement device of this disclosure can have a higher detecting sensitivity.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A pressure sensor, comprising:
a case having a concave portion and a convex portion disposed at two opposite sides of the case, respectively, wherein a recessed space is formed in the concave portion; and
a pressure sensing element disposed on the case, wherein when the convex portion pushes the pressure sensing element, the pressure sensing element is deformed and protruded toward the recessed space.

2. The pressure sensor according to claim 1, wherein the pressure sensing element is a strain gauge or a piezoelectric element.

3. The pressure sensor according to claim 1, wherein the concave portion, the convex portion and the pressure sensing element are at least partially overlapped in a projection direction.

4. The pressure sensor according to claim 1, wherein the convex portion has an arc surface, and the pressure sensing element fits to the arc surface.

5. The pressure sensor according to claim 1, wherein the concave portion has an arc surface, and the pressure sensing element fits to the arc surface.

6. The pressure sensor according to claim 1, wherein the pressure sensing element is disposed inside the case and located between the concave portion and the convex portion.

7. The pressure sensor according to claim 1, wherein a side wall of the concave portion has a stepped shape.

8. The pressure sensor according to claim 1, wherein a plurality of ribs are disposed inside the concave portion, and the ribs are intersected to form a plurality of the recessed spaces.

9. A blood pressure measurement device, comprising:
a pressure sensor according to claims 1 to 8; and
a first electrode disposed on a surface of the case away from the convex portion.

10. The blood pressure measurement device according to claim 9, wherein the concave portion is disposed inside the case and located between the concave portion and the surface.

11. The blood pressure measurement device according to claim 9, wherein the blood pressure measurement device is operated on a body portion of a user having a blood vessel, and when measuring a blood pressure, the first electrode, the concave portion, the pressure sensing element, the convex portion and the blood vessel are at least partially overlapped in a projection direction.

12. The blood pressure measurement device according to claim 11, wherein when the user touches the first electrode, the user pushes the convex portion to fit on the body portion.

13. The blood pressure measurement device according to claim 12, wherein a finger of the user is contact with the first electrode, the case has a recess corresponding to a shape of the finger, and the first electrode is disposed in the recess.

14. The blood pressure measurement device according to claim 11, further comprising:
a second electrode disposed adjacent to the convex portion and electrically connected to the first electrode, wherein when measuring the blood pressure, the second electrode is contacted with the body portion.

15. The blood pressure measurement device according to claim 14, further comprising:
a calibration electrode disposed on the case;
a processing unit disposed in the case; and
a storage unit disposed in the case and signally connected to the processing unit, wherein the storage unit stores a program instruction, and when the processing unit executes the program instruction, the processing unit performs following steps of:
obtaining a first calibration value, wherein the first calibration value is obtained by calculating a first measurement result of the calibration electrode and the first electrode;
obtaining a second calibration value, wherein the second calibration value is obtained by calculating a second measurement result of the calibration electrode and the first electrode; and
calibrating measured values of the first electrode and the second electrode according to the first calibration value and the second calibration value.
